# EUROPEAN PATENT SPECIFICATION

(11) **EP 3 398 454 B1**
(45) Date of publication and mention of the grant of the patent: **21.07.2021**
(21) Application number: 18174303.0
(22) Date of filing: 19.10.2012
(51) Int. Cl.: A23L 33/19, A23L 33/00, A61K 35/20, A61P 21/06

(54) **WHEY PROTEIN MICELLES AGAINST MUSCLE ATROPHY AND SARCOPENIA**
MOLKEPROTEINMIZELLEN GEGEN MUSKELATROPHIE UND SARKOPENIE
MICELLES DE PROTÉINES DE LACTOSÉRUM CONTRE L'AMYOTROPHIE ET LA SARCOPÉNIE

(30) Priority: 21.10.2011 EP 11186143
(43) Date of publication of application: 07.11.2018
(62) Divisional of application: 12773336.8
(73) Proprietor: Société des Produits Nestlé S.A., 1800 Vevey (CH)
(72) Inventor: BREUILLE, Denis, 1010 Lausanne (CH); MOORE, Daniel, Waterdown, Ontario L0R 2H3 (CA); STELLINGWERFF, Trent, Victoria, British Columbia V8YIX8 (CA); POUTEAU, Etienne, 75013 PARIS (FR); BOVETTO, Lionel Jean René, 1522 LUCENS (CH)
(74) Representative: Chautard, Cécile

(56) References cited:
- WO-A1-2011/011252
- WO-A1-2011/112695
- WO-A2-2006/034857
- US-A- 5 882 705
- US-A1- 2011 250 310
- ACHESON K J ET AL: "Protein choices targeting thermogenesis and metabolism", THE AMERICAN JOURNAL OF CLINICAL NUTRITION, AMERICAN SOCIETY FOR NUTRITION, US, vol. 93, no. 3, 1 January 2011 (2011-01-01), pages 525-534, XP009156726, ISSN: 0002-9165

## Description

The present invention relates to whey protein micelles for use in the treatment and/or prevention of a condition linked to a reduced concentration of plasma amino acids in a patient. A further aspect of the invention is a meal replacement comprising whey protein micelles.

The loss of muscle mass and muscle strength considerably decreases the quality of life of a patient suffering from such a condition as he becomes unable to perform certain physical tasks and the risk of accidents related to such physical tasks like for example walking becomes increased. One may distinguish two major conditions which lead to a loss of muscle mass and strength, one being muscle atrophy and the other being sarcopenia. Muscle atrophy results from co-morbidity of several common diseases, including cancer, AIDS, congestive heart failure, chronic obstructive pulmonary disease and others. Disuse of the muscles from a lack of physical exercise for a longer period of time will also lead to muscle atrophy. Thereby, particularly bedridden patients can have significant muscle wasting. Moreover, starvation eventually leads to muscle atrophy as can be observed for example with overweight patients on a strict weight-loss diet. Sarcopenia relates to the gradual decrease in the ability of maintaining muscle mass and strength which comes with age.

Loss of muscle mass occurs by a change in the normal balance between protein synthesis and protein degradation. During atrophy, for example, there is a down-regulation of protein synthesis pathways, and an activation of protein breakdown pathways (Sandri M, 2008, Physiology 23:160-170). Since the absence of muscle-building amino acids, particularly of branched chain amino acids, can contribute to muscle wasting, the provision of sufficient amino acids can be helpful in regenerating damaged or atrophied muscle tissue. The branched chain amino acids (BCAAs), including leucine, isoleucine and valine, are critical in this process. Thereby, nutrition leading to a sustained hyper-aminoacidemia, i.e. an elevated concentration of amino acids in the plasma, especially of the BCAAs and further essential amino acids, is essential in stimulating muscle protein synthesis of a patient in need.

Previous studies demonstrated that an ingestion of a mixed meal typically stimulates skeletal muscle protein synthesis and that an adequate supply of amino acids is essential. Thereby, recent studies suggest that it is the supply of BCAAs and particulary of leucine, that modulate the protein synthetic response in skeletal muscle to meal feeding (Garlick PJ et al., 1988, Biochem J 254:579-584; Anthony JC et al., 1999, J Nutr 129:1102-1106; Crozier SJ et al., 2005, J Nutr 135:376-382). Further research indicated that the leucine content of a selected protein source of a meal is an important indicator of the protein quality as it relates to acute stimulation of muscle protein synthesis (Norton LE et al., 2009, J Nutr 139:1103-1109).

Tang JE et al. (2009, J Appl Physiol 107:987-992) investigated the response of skeletal muscle protein synthesis in young men following the ingestion of three distinct but high-quality dietary proteins, i.e. whey, micellar casein and soy, at rest and after resistance exercise. Thereby, it was reported that the consumption of whey proteins stimulated muscle protein synthesis to a greater degree than casein, both at rest and after resistance exercise. Whey proteins stimulated also a significantly larger rise in muscle synthesis than soy proteins, which was in congruence with previous work of the same authors. They concluded that whey proteins stimulate skeletal muscle protein synthesis to a greater extent than either casein or soy proteins, both at rest and after resistance exercise.

In accordance with this, WO2011/112695 lists a number of health benefits of whey proteins, among them enhancement of muscle development and building, as well as muscle maintenance in children, adults or elderly people.

Also US2011/250310 discloses that a whey composition combined with active ingredients such as vitamin D can help to improve muscular-skeletal health in elderly persons.

WO2011/011252 discloses a method of attenuating the loss of functional status comprising a nutritional intervention that helps prevent the loss of muscle mass, said nutritional invention comprising - next to many other active ingredients - whey protein; and an exercise regimen.

There is still a persisting need in the food industry to find better nutritional solutions for patients suffering from a loss of muscle mass or muscle strength. In particular for subjects that are unable to perform exercise regimens, a nutritional solution would be needed that is effective on its own.

The object of the present invention is to improve the state of the art and to provide a nutritional solution that addresses the needs expressed above and that helps to maintain an elevated concentration of plasma amino acids in a patient in need thereof.

The object of the present invention is achieved by the subject matter of the independent claims. The dependent claims further develop the idea of the present invention.

Accordingly, the present invention provides in a first aspect whey protein micelles for use in the treatment and/or prevention of a condition linked to a reduced concentration of plasma amino acids in a patient in need thereof, wherein the treatment or prevention does not include an additional exercise regimen, wherein the condition is muscle atrophy or sarcopenia, wherein the whey protein micelles are administered to the patient in combination with a meal, wherein the meal comprises whey protein isolates, hydrolysed milk proteins or any combination thereof; and wherein the whey protein micelles are administered to the patient in a daily dose of at least 20 g dry weight.

That treatment or prevention does not require an additional exercise regimen to be effective. Hence, the treatment or prevention does not include an additional exercise regimen.

In a second aspect, the invention relates to a meal replacement comprising whey protein micelles, further comprising whey protein isolates, hydrolysed milk proteins or any combination thereof, wherein the whey protein micelles are present in said meal replacement in an amount of at least 15% of total dry weight.

"Whey protein micelles" (WPM) are defined herein as described in EP1839492A1 and as further characterized in Schmitt C et al. (2010, Soft Matter 6:4876-4884), where they are referred to as whey protein microgels (WPM). Particularly, the "whey protein micelles" are the micelles comprised in the whey protein micelles concentrate obtainable by the process as disclosed in EP1839492A1. Therein, the process for the production of whey protein micelles concentrate comprises the steps of: a) adjusting the pH of a whey protein aqueous solution to a value between 3.0 and 8.0; b) subjecting the aqueous solution to a temperature between 80 and 98°C; and c) concentrating the dispersion obtained in step b). Thereby, the micelles produced have an extremely sharp size distribution, such that more than 80% of the micelles produced have a size smaller than 1 micron in diameter and preferably are between 100 nm and 900 nm in size. The "whey protein micelles" can be in liquid concentrate or in powder form. Importantly, the basic micelle structure of the whey proteins is conserved, in the concentrate, the powder and reconstituted from the powder for example in water. The "whey protein micelles" are physically stable in dispersion, as powder as well as during spray-drying or freeze-drying.

A rapid increase in plasma amino acids is required for stimulating muscle protein synthesis at rest and after exercise (Dangin M et al., 2003, J Physiol 549:635-644). One of the currently best solutions for providing this rapid increase in plasma amino acids is whey protein isolate (WPI) (Tang JE et al., 2009, J Appl Physiol 107:987-992). A more sustained amino acid response may prolong the anabolism and increase muscle protein synthesis by providing amino acid building blocks over a longer period of time (Lacroix M et al., 2006: Am J Clin Nutr 84:107-9). In addition, a more slowly digested protein may suppress protein breakdown (Dangin M et al., 2001, Am J Physiol 280:E340-E348), which would have an additional benefit for the net muscle protein balance, i.e. the difference between protein synthesis and protein breakdown. Thus, a protein or a mix of proteins that would induce the maximal aminoacidemia but during a longer period of time would do both, i.e. maximally stimulate protein synthesis and suppress protein breakdown.

It has now been surprisingly found by the inventors that whey protein micelles consumed as part of a meal induce the same high plasma aminoacidemia as an iso-caloric and iso-nitrogenous control meal with whey protein isolates (WPI), but significantly delayed postprandially by about 30 min with respect to that of the control meal. Consequently, surprisingly, the micelle structure generates an improved health benefit as compared to normal whey protein. To the inventors best knowledge this has never been reported. The peak amino acid concentration (i.e. Cmax) after the whey protein micelles meal was the same as after the WPI meal, and significantly higher than the maximum concentrations reached after an iso-caloric and iso-nitrogenous milk protein or milk casein meal. The results of the clinical study are presented in the Example section.

Hence, the inventors have found a protein composition which when consumed as part of a regular meal induces a delayed but high maximal aminoacidemia in a subject. This hyper-aminoacidemia for a prolonged postprandial period of time is most favourable for maximally stimulating muscle protein synthesis, reducing protein breakdown and therefore maintaining or even enhancing muscle mass.

"Hyper-aminoacidemia" is an excess of amino acids in the bloodstream, the amino acid pool, which can lead to an increase in protein synthesis and reduction of protein breakdown with an overall positive nitrogen balance. Thereby, the positive nitrogen balance indicates more construction of lean tissue than destruction, leading overall to an increase in lean body mass.

Although not wishing to be bound by theory, the inventors think that whey protein micelles as part of a meal seem to induce a delayed gastric emptying or to be more slowly digested as compared to native whey proteins such as WPI.

Thereby, whey protein micelles deliver the amino acids more slowly into the peripheral blood circulation.
Figure 1: Plasma concentrations of essential amino acids 3 h after the ingestion of meal replacements comprising whey protein isolate, whey protein micelles or micellar casein.
Figure 2: Plasma concentrations of leucine 3 h after the ingestion of meal replacements comprising whey protein isolate, whey protein micelles or micellar casein.
Figure 3: Plasma concentrations of essential amino acids 3 h after the ingestion of meal replacements comprising each one of the 7 different proteins.

The present invention pertains to whey protein micelles for use in the treatment and/or prevention of a condition linked to a reduced concentration of plasma amino acids in a patient, wherein the condition is linked to a loss of muscle mass and/or strength. The hyper-aminoacidemia for a prolonged postprandial period of time provided by the inventive use of the whey protein micelles is most favourable for maximally stimulating muscle protein synthesis and therefore maintaining or even enhancing muscle mass.

According to the present invention, the condition is muscle atrophy or sarcopenia. Both medical conditions are characterized by a loss of muscle mass and strength. The present invention is best adapted to providing a nutritional solution to patients suffering from either of those conditions, to reduce or stop loss of muscle mass and/or ultimately to build up again muscle mass and strength.

"Muscle atrophy" is defined as a decrease in the mass of muscles in a subject. It can be a partial or complete wasting away of muscle tissue. When a muscle atrophies, this leads to muscle weakness, since the ability to exert force is related to muscle mass. Muscle atrophy results from a co-morbidity of several common diseases, including cancer, AIDS, congestive heart failure and chronic obstructive pulmonary disease. Moreover, starvation eventually leads to muscle atrophy. Disuse of the muscles will also lead to atrophy.

"Sarcopenia" is defined as the degenerative loss of skeletal muscle mass and strength associated with aging. Sarcopenia is characterized first by a decrease in the size of the muscle, which causes weakness and frailty. However, this loss of muscle mass may be caused by different cellular mechanisms than those that cause muscle atrophy. For example, during sarcopenia, there is a replacement of muscle fibres with fat and an increase in fibrosis.

The whey protein micelles for use according to the invention particularly pertains to a patient, who is a critically ill patient, a patient after surgery, a trauma patient, a cancer patient, an overweight person during weight-loss dieting or a patient during and after bed rest. The common fate of all these patients is that they are dramatically losing muscle mass and/or are at risk of dramatically losing (even further) muscle mass. Hence, it is those patients that would maximally profit from the new current invention.

A "critically ill patient" is defined as a patient who is at high risk for an actual or potential life-threatening health problem. The more critically ill the patient is the more likely he or she is to be highly vulnerable, unstable and complex, thereby requiring intense and vigilant nursing care.

A "trauma patient" is a person who has suffered a trauma. Thereby, trauma refers to a body wound or shock produced by sudden physical injury, as for example from violence or an accident. People who have suffered trauma usually require specialized care.

A "cancer patient" is a patient who has cancer.

An "overweight person during weight-loss dieting": Overweight people, or people suffering from obesity, typically aim to lose weight and fat by following a diet. Normally, when people lose weight, they lose a combination of fat and muscle. Thereby, a severe and prolonged diet can lead to a significant loss of muscle mass affecting strength and metabolism. Therefore, maintaining muscle mass while losing fat is a key factor to reach both, the ideal weight and body composition.

A "patient during and after bed rest": Disuse atrophy occurs in a patient from a lack of physical exercise. Thereby, the muscle atrophy is caused by not using the muscles enough. People with medical conditions that limit their movement or their physical activity as it is for example the case for bedridden patients can lose muscle mass and strength.

According to the invention, the whey protein micelles for use according to the invention are administered to the patient in combination with a meal. Most meals comprise proteins from a milk, plant and/or animal source and hence upon consumption lead to a postprandial aminoacidemia increase, i.e. an elevated concentration of amino acids in the plasma of the consumer. It is now an advantage, to combine the administration of whey protein micelles in combination with such a meal. Thereby, the postprandial plasma amino acid peak resulting from the proteins present in the meal adds up to the postprandial amino acid peak resulting from the whey protein micelles which are delayed by ca. 30 min in respect to the first amino acid peak. Thereby, the overall resulting hyper-aminoacidemia is extended and prolonged in time. This in return is most favourable for maximally stimulating muscle protein synthesis, reducing muscle protein breakdown and therefore maintaining or even enhancing muscle mass.

In a preferred embodiment, the meal comprises whey protein isolates, native or hydrolyzed milk proteins, free amino acids, or a combination thereof. As known from earlier studies, a whey protein meal exhibits a significantly stronger aminoacidemia effect on subjects than for example a plant protein meal. Therefore, advantageously, the whey protein micelles are combined with a meal comprising whey proteins in the form of WPI or milk. Advantageously, the meal can be even further supplemented with free amino acids in combination with the whey or milk proteins to optimally induce a hyper-aminoacidemia upon consumption of said meal.

The whey protein micelles for use according to the invention is to be administered to the patient during a period of at least one day before surgery and/or hospital stay to at least one week after surgery and/or hospital stay. Thereby, advantageously, a patient builds up his plasma amino acid pool already before undergoing surgery or a longer bedridden hospital stay and continues to maintaining such an elevated concentration of the essential amino acids during the full period of recovery. This provides him with an optimal nutritional status to minimize loss of muscle mass during the hospital intervention and also prepares him for a quicker recovery and build up of lost muscle tissues thereafter.

In a preferred embodiment, the whey protein micelles are administered to a subject in a daily dose of at least 20 g dry weight, preferably of at least 30 g dry weight. Those doses should assure a sufficient daily quantity for providing the desired effect to a subject in at least a mid-term period.

In a particular embodiment, the whey protein micelles are provided in the form of a liquid meal replacement. Whey protein micelles have the advantage of having a significantly better solubility in water than for example whey protein isolates (WPI). Thereby, about twice the amount of whey proteins can be solubilized and provided in a liquid meal replacement form in comparison to a WPI based liquid meal. This confers a significant advantage and originality for the production of liquid meal replacers and meal replacement systems. It allows a.o. also to provide liquid meal replacement products with high amounts of whey proteins for applications in e.g. enteral nutrition feeding.

In a further aspect, the invention relates to a meal replacement comprising whey protein micelles which further comprises whey protein isolates, hydrolyzed milk proteins, free amino acids or any combination thereof.

As indicated above, it is of an advantage to combine the administration of whey protein micelles with whey proteins in the form of WPI, milk and/or even free amino acids to optimally induce and extend a hyper-aminoacidemia upon consumption of such a meal. Preferably, the different protein components are combined together into one meal replacement product or kit of products. Thereby, the individual protein components can be optimally dosed for providing a best and prolonged hyper-aminoacidemia effect and at the same time optimized for a good, organoleptically best acceptable product application.

According to the invention, the whey protein micelles are present in a meal replacement in an amount of at least 15 wt%, preferably of at least 20 wt% of total dry weight.

In a preferred embodiment, the meal replacement according to the invention comprises 15-50 wt% proteins, 10-15 wt% lipids, 25-50 wt% carbohydrates and 5-10 wt% fibers of total dry weight of the meal replacement.

The meal replacement can be provided in liquid form. It can also be provided in a form suitable for enteral tube feeding. Those skilled in the art will understand that they can freely combine all features of the present invention disclosed herein. In particular, features described for the therapeutic use may be used and combined with the features of the meal replacement product, and vice versa. Further, features described for different embodiments of the present invention may be combined.

Further advantages and features of the present invention are apparent from the figures and examples.

### Example

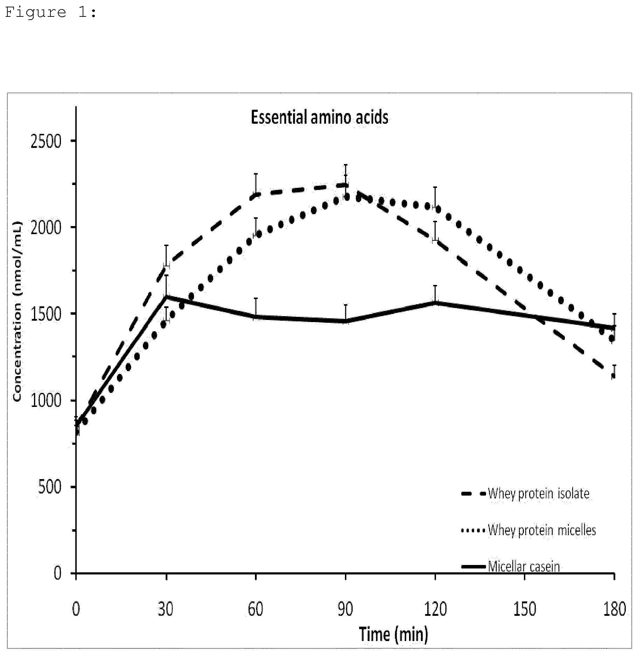

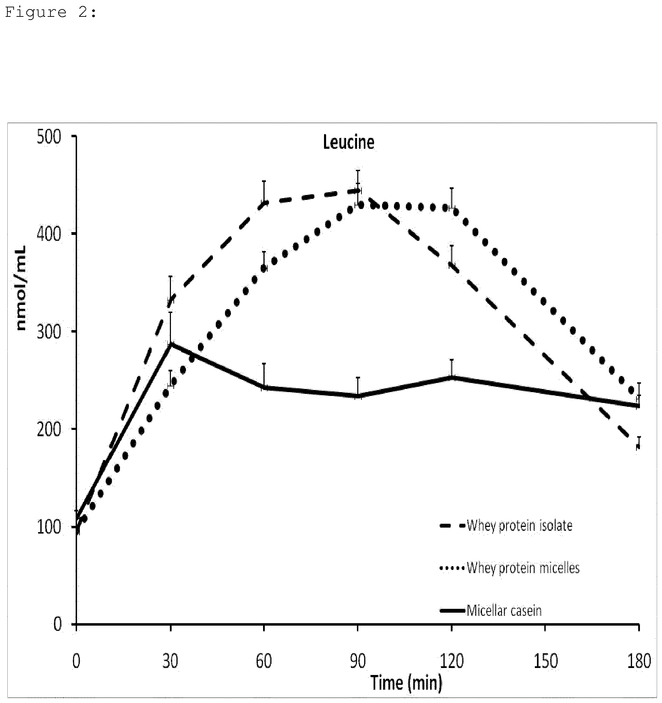

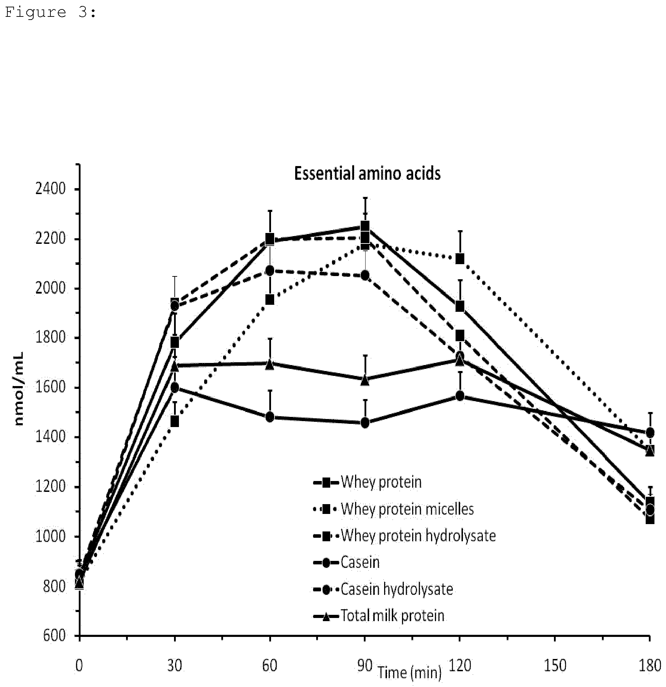

A randomized double-blind 7-arm crossover study was performed in twenty-three healthy men in the following way. A test meal replacement was ingested at lunch time on 7 separate occasions separated each by a wash-out period of one week. The meal replacements were iso-caloric and iso-nitrogenous. They were composed of the tested protein (30g, 7.2% w/w), lipids (11.7g, 2.8% w/w), carbohydrates (42.7g, 10.2% w/w) and fibers (6.3g, 1.5% w/w). The tested proteins were: (1) whey protein isolate (WPI); (2) whey protein micelles (WPM); (3) extensively hydrolyzed whey protein (EHWP); (4) micellar casein (ICP); (5) extensively hydrolyzed casein protein (EHCP); (6) total milk proteins (TMP); and (7) extensively hydrolyzed milk proteins (EHMP). The meal replacements were completed with water to 430mL and contained 388kcal per serving.
Arterialized venous blood samples were taken, via a catheter inserted into a wrist vein of the volunteers, before and for 3h after consuming the test meal replacement. Plasma samples were used to analyze amino acids by gas chromatography and mass spectrometry. The results are shown in Figures 1 to 3.

Firstly, the results confirmed that intact whey protein induces a higher aminoacidemia than micellar casein. Secondly, it was found that the peaks of the postprandial plasma amino acid concentrations after consumption of the WPI and WPM test meal replacements, although similar in extent and height, were delayed by approximately 30 min, i.e. occurring at 120 min rather than at 90 min. This allowed maintenance of an elevated concentration of plasma amino acids for a prolonged period of time after the ingestion of the whey protein micelles (Figures 1 to 3: small dotted lines).

## Claims

1. Whey protein micelles for use in the treatment and/or prevention of a condition linked to a reduced concentration of plasma amino acids in a patient in need thereof, wherein the treatment or prevention does not include an additional exercise regimen, wherein the condition is muscle atrophy or sarcopenia and wherein the whey protein micelles are administered to the patient in combination with a meal, wherein the meal comprises whey protein isolates, hydrolyzed milk proteins or any combination thereof; and wherein the whey protein micelles are administered to the patient in a daily dose of at least 20 g dry weight.

2. The whey protein micelles for use according to claim 1, wherein the condition is linked to a loss of muscle mass and/or strength.

3. The whey protein micelles for use according to one of the preceding claims, wherein the patient is a critically ill patient, a patient after surgery, a trauma patient, a cancer patient, an overweight person during weight-loss dieting or a patient during and after bed rest.

4. The whey protein micelles for use according to claim 1, wherein the meal further comprises whey protein isolates, hydrolyzed milk proteins, free amino acids or any combination thereof.

5. The whey protein micelles for use according to one of the preceding claims, to be administered to the patient during a period of at least one day before surgery and/or hospital stay to at least one week after surgery and/or hospital stay.

6. The whey protein micelles for use according to one of the preceding claims, wherein said use comprises the administration to the patient in a daily dose of at least 30 g dry weight.

7. The whey protein micelles for use according to one of the preceding claims, provided in the form of a liquid meal replacement.

8. A meal replacement comprising whey protein micelles further comprising whey protein isolates, hydrolyzed milk proteins or any combination thereof, wherein the whey protein micelles are present in said meal replacement in an amount of at least 15 wt%. of total dry weight

9. The meal replacement according to claim 8, further comprising free amino acids.

10. The meal replacement according to claim 8 or 9, wherein the whey protein micelles are present in said meal replacement in an amount of at least 20 wt% of total dry weight.

11. The meal replacement according to one of the claims 8-10, comprising 25-50 wt% proteins, 10-15 wt% lipids, 25-50 wt% carbohydrates and 5-10 wt% fibers of total dry weight.

12. The meal replacement according to one of the claims 8-11, provided in liquid form.

13. The meal replacement according to one of the claims 8-12, provided in a form suitable for enteral tube feeding.

## Patentansprüche

1. Molkeproteinmizellen zur Anwendung bei der Behandlung und/oder Prävention eines Zustands im Zusammenhang mit einer reduzierten Konzentration von Plasmaaminosäuren bei einem Patienten, der diese benötigt, wobei die Behandlung oder Prävention kein zusätzliches Übungsprogramm einschließt, wobei der Zustand Muskelatrophie oder Sarkopenie ist und wobei die Molkeproteinmizellen dem Patienten in Kombination mit einer Mahlzeit verabreicht werden, wobei die Mahlzeit Molkeproteinisolate, hydrolysierte Milchproteine oder eine beliebige Kombination davon umfasst; und wobei die Molkeproteinmizellen dem Patienten in einer Tagesdosis von mindestens 20 g Trockenmasse verabreicht werden.

2. Molkeproteinmizellen zur Anwendung nach Anspruch 1, wobei der Zustand mit einem Verlust von Muskelmasse und/oder -kraft verbunden ist.

3. Molkeproteinmizellen zur Anwendung nach einem der vorstehenden Ansprüche, wobei der Patient ein schwer kranker Patient, ein Patient nach einer Operation, ein Traumapatient, ein Krebspatient, eine Person mit Übergewicht während einer Gewichtsreduktionsdiät oder ein Patient während und nach der Bettruhe ist.

4. Molkeproteinmizellen zur Anwendung nach Anspruch 1, wobei die Mahlzeit ferner Molkeproteinisolate, hydrolysierte Milchproteine, freie Aminosäuren oder eine Kombination davon umfasst.

5. Molkeproteinmizellen zur Anwendung nach einem der vorstehenden Ansprüche, die einem Patienten während des Zeitraums von mindestens einem Tag vor der Operation und/oder dem Krankenhausaufenthalt bis zu mindestens einer Woche nach der Operation und/oder dem Krankenhausaufenthalt zu verabreichen sind.

6. Molkeproteinmizellen zur Anwendung nach einem der vorstehenden Ansprüche, wobei diese Anwendung die Verabreichung an den Patienten in einer Tagesdosis von mindestens 30 g Trockenmasse umfasst.

7. Molkeproteinmizellen zur Anwendung nach einem der vorstehenden Ansprüche, die in Form eines flüssigen Mahlzeitersatzes bereitgestellt sind.

8. Mahlzeitersatz, umfassend Molkeproteinmizellen, ferner umfassend Molkeproteinisolate, hydrolysierte Milchproteine oder eine beliebige Kombination davon, wobei die Molkeproteinmizellen in dem Mahlzeitersatz in einer Menge von mindestens 15 Gew.-% der Gesamttrockenmasse vorhanden sind.

9. Mahlzeitersatz nach Anspruch 8, ferner umfassend freie Aminosäuren.

10. Mahlzeitersatz nach Anspruch 8 oder 9, wobei die Molkenproteinmizellen in dem Mahlzeitersatz in einer Menge von mindestens 20 Gew.-% der Gesamttrockenmasse vorhanden sind.

11. Mahlzeitersatz nach einem der Ansprüche 8-10, umfassend 25-50 Gew.-% Proteine, 10-15 Gew.-% Lipide, 25-50 Gew.-% Kohlenhydrate und 5-10 Gew.-% Fasern der Gesamttrockenmasse.

12. Mahlzeitersatz nach einem der Ansprüche 8-11, der in flüssiger Form bereitgestellt wird.

13. Mahlzeitersatz nach einem der Ansprüche 8-12, der in einer für enterale Sondennahrung geeigneten Form bereitgestellt wird.

## Revendications

1. Micelles de protéines de lactosérum pour utilisation dans le traitement et/ou la prévention d'une condition liée à une concentration réduite d'acides aminés plasmatiques chez un patient qui en a besoin, dans lesquelles le traitement ou la prévention n'inclut pas de programme d'exercice supplémentaire, dans lesquelles la condition est une atrophie musculaire ou sarcopénie et dans lesquelles les micelles de protéines de lactosérum sont administrées au patient en combinaison avec un repas, dans lesquelles le repas comprend des isolats de protéines de lactosérum, des protéines de lait hydrolysées ou n'importe quelle combinaison de ceux-ci ; et dans lesquelles les micelles de protéines de lactosérum sont administrées au patient à une dose journalière d'au moins 20 g de poids sec.

2. Micelles de protéines de lactosérum pour utilisation selon la revendication 1, dans lesquelles la condition est liée à une perte de masse et/ou force musculaires.

3. Micelles de protéines de lactosérum pour une utilisation selon l'une des revendications précédentes, dans lesquelles le patient est un patient gravement malade, un patient après une intervention chirurgicale, un patient souffrant de traumatisme, un patient cancéreux, une personne en surpoids pendant un régime amaigrissant ou un patient pendant et après un alitement.

4. Micelles de protéines de lactosérum pour utilisation selon la revendication 1, dans lesquelles le repas comprend en outre des isolats de protéines de lactosérum, des protéines de lait hydrolysées, des acides aminés libres ou n'importe quelle combinaison de ceux-ci.

5. Micelles de protéines de lactosérum pour utilisation selon l'une des revendications précédentes, à administrer au patient pendant une période d'au moins un jour avant intervention chirurgicale et/ou séjour à l'hôpital jusqu'à au moins une semaine après intervention chirurgicale et/ou séjour à l'hôpital.

6. Micelles de protéines de lactosérum pour utilisation selon l'une des revendications précédentes, dans lesquelles ladite utilisation comprend l'administration au patient en une dose journalière d'au moins 30 g de poids sec.

7. Micelles de protéines de lactosérum pour utilisation selon l'une des revendications précédentes, fournies sous la forme d'un substitut de repas liquide.

8. Substitut de repas comprenant des micelles de protéines de lactosérum comprenant en outre des isolats de protéines de lactosérum, des protéines de lait hydrolysées ou n'importe quelle combinaison de ceux-ci, dans lequel les micelles de protéines de lactosérum sont présentes dans ledit substitut de repas en une quantité d'au moins 15 % en poids du poids sec total

9. Substitut de repas selon la revendication 8, comprenant en outre des acides aminés libres.

10. Substitut de repas selon la revendication 8 ou 9, dans lequel les micelles de protéines de lactosérum sont présentes dans ledit substitut de repas en une quantité d'au moins 20 % en poids du poids sec total.

11. Substitut de repas selon l'une des revendications 8 à 10, comprenant 25 à 50 % en poids de protéines, 10 à 15 % en poids de lipides, 25 à 50 % en poids de glucides et 5 à 10 % en poids de fibres du poids sec total.

12. Substitut de repas selon l'une des revendications 8 à 11, fourni sous forme liquide.

13. Substitut de repas selon l'une des revendications 8 à 12, fourni sous une forme appropriée pour alimentation par sonde entérale.
